(19) Europäisches Patentamt
European Patent Office
Office européen des brevets

(11) Veröffentlichungsnummer: **0 041 673**
**B1**

(12) # EUROPÄISCHE PATENTSCHRIFT

(45) Veröffentlichungstag der Patentschrift: **10.10.84**

(51) Int. Cl.[3]: **C 07 D 405/12,** A 61 K 31/415

(21) Anmeldenummer: **81104168.0**

(22) Anmeldetag: **01.06.81**

(54) **Imidazolderivate, deren Herstellung sowie diese enthaltende Präparate.**

(30) Priorität: **06.06.80 GB 8018691**
**02.03.81 GB 8106459**

(43) Veröffentlichungstag der Anmeldung: **16.12.81 Patentblatt 81/50**

(45) Bekanntmachung des Hinweises auf die Patenterteilung: **10.10.84 Patentblatt 84/41**

(84) Benannte Vertragsstaaten: **AT BE CH DE FR GB IT LI LU NL SE**

(56) Entgegenhaltungen:
**DE - A - 2 619 381**
**DE - A - 2 633 492**
**DE - A - 2 917 244**
**US - A - 4 045 568**

(73) Patentinhaber: **F. HOFFMANN-LA ROCHE & CO. Aktiengesellschaft, CH-4002 Basel (CH)**

(72) Erfinder: **Martin, Joseph Armstrong, 82 Chesterton Avenue, Harpenden, Herts. (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft Imidazolderivate, deren Herstellung und pharmazeutische Präparate auf deren Basis.

Bei den Imidazolderivaten der vorliegenden Erfindung handelt es sich um Verbindungen der allgemeinen Formel

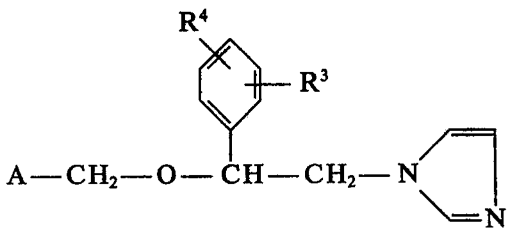

(I)

worin A eine Gruppe der Formeln

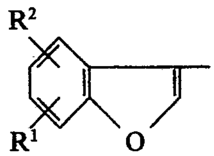

oder

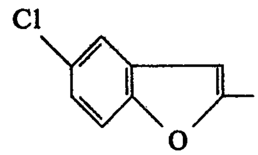

(a) (b)

und $R^1$, $R^2$, $R^3$ und $R^4$ Wasserstoff oder Halogen darstellen,
und um deren pharmazeutisch verträgliche Säureadditionssalze.

Der Ausdruck »Halogen« umfaßt Fluor, Chlor, Brom und Jod.

Der Stand der Technik wird illustriert durch die deutschen Offenlegungsschriften Nr. 2 619 381, Nr. 2 633 492 und Nr. 2 819 244, sowie durch die US-Patentschrift Nr. 4 045 568, welche Imidazolderivate mit antimykotischer Wirkung beschreiben.

Die erfindungsgemäßen Verbindungen unterscheiden sich strukturell von diesen bekannten Imidazolderivaten, indem sie einen Benzofuranyl-methoxy Rest enthalten.

Eine bevorzugte Gruppe von erfindungsgemäßen Imidazolderivaten sind solche der Formel I mit A = Gruppe der Formel (a), $R^1$ = Wasserstoff oder Halogen und $R^2$, $R^3$ und $R^4$ = Halogen, vorzugsweise Chlor. Besonders bevorzugte Imidazolderivate dieser Gruppe sind:

1-[2,4-Dichlor-$\beta$-[(4,6-dichlorbenzofuran-3-yl)-methoxy]phenethyl]imidazol,
1-[2,4-Dichlor-$\beta$-[(5,7-dichlorbenzofuran-3-yl)-methoxy]phenethyl]imidazol,
1-[2,4-Dichlor-$\beta$-[(6-chlorbenzofuran-3-yl)-methoxy]phenethyl]imidazol

und deren pharmazeutisch verträgliche Säureadditionssalze.

Eine andere Gruppe bevorzugter Imidazolderivate sind solche der Formel I mit A = Gruppe der Formel (b) und $R^3$ und $R^4$ = Halogen, vorzugsweise Chlor. Eine besonders bevorzugte Verbindung dieser Gruppe ist:

1-[2,4-Dichlor-$\beta$-[(5-chlorbenzofuran-2-yl)methoxy]phenethyl]imidazol

und deren pharmazeutisch verträgliche Säureadditionssalze.

Die Imidazolderivate I und ihre pharmazeutisch verträglichen Säureadditionssalze werden erfindungsgemäß dadurch hergestellt, daß man eine Verbindung der Formel

$$A-CH_2-X \qquad (II)$$

worin A die obengenannte Bedeutung hat und X Chlor oder Brom darstellt,

mit einem Alkalimetallsalz eines Alkohols der allgemeinen Formel

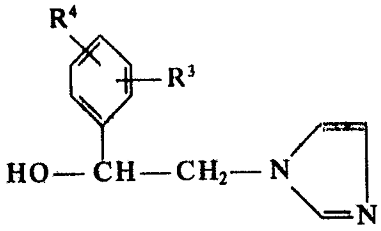

(III)

worin $R^3$ und $R^4$ die obengenannten Bedeutungen haben,

umsetzt, und, gewünschtenfalls, ein erhaltenes Racemat in die optischen Isomeren auftrennt sowie, gewünschtenfalls, eine erhaltene Verbindung I in ein pharmazeutisch verträgliches Säureadditionssalz überführt.

Die Umsetzung einer Verbindung der Formel II, vorzugsweise mit X = Brom, mit einem Alkalimetallsalz, vorzugsweise dem Natriumsalz eines Alkohols der Formel III, wird zweckmäßigerweise in Gegenwart eines inerten organischen Lösungsmittels durchgeführt. Dimethylformamid ist ein bevorzugtes Lösungsmittel, obgleich andere Lösungsmittel, wie aromatische Kohlenwasserstoffe (z. B. Benzol, Toluol), Ether (z. B. 1,2-Dimethoxyethan, Tetrahydrofuran) usw., ebenfalls verwendet werden können. Die Umsetzung kann bei einer Temperatur zwischen etwa 0°C und der Rückflußtemperatur des Reaktionsgemisches, vorzugsweise bei einer Temperatur zwischen 0°C und Raumtemperatur, durchgeführt werden. In einer bevorzugten Ausführungsform wird das Alkalimetallsalz eines Alkohols der Formel III in situ hergestellt durch Umsetzung eines Alkohols der Formel III mit einer geeigneten Base wie einem Alkalimetallhydrid (z. B. Natriumhydrid) oder einem Alkalimetallamid (z. B. Natriumamid).

Die Ausgangsverbindungen der Formel II sind bekannte Verbindungen oder Analoge von bekannten Verbindungen. Die Ausgangsmaterialien der Formel II mit A = Gruppe der Formel (a) können beispielsweise gemäß folgendem Reaktionsschema, in dem $R^1$, $R^2$ und X die obengenannten Bedeutungen haben, hergestellt werden:

Formelschema

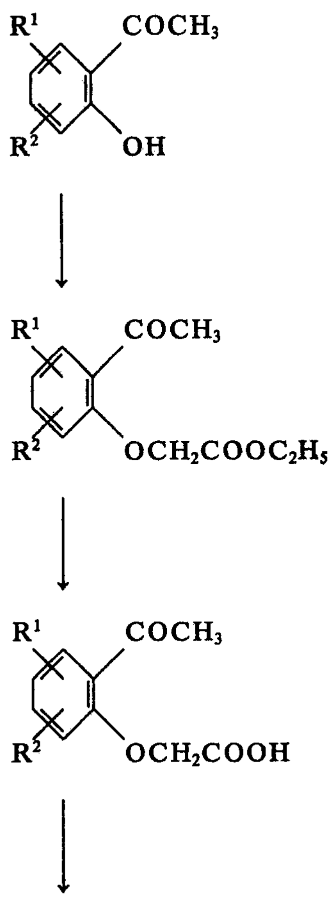

(IV)

(V)

(VI)

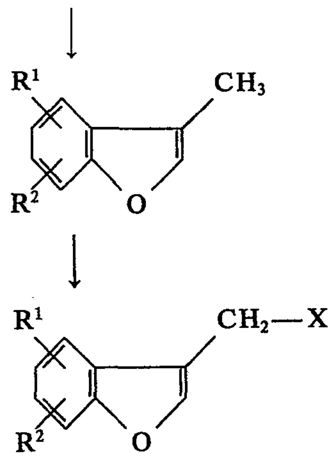

(VII)

(IIa)

Gemäß Reaktionsschema wird in einer ersten Stufe ein Phenol der Formel IV in an sich bekannter Weise in eine Verbindung V überführt, beispielsweise durch Umsetzung mit Bromessigsäureethylester in Gegenwart einer Base wie Kaliumcarbonat und in Gegenwart eines inerten organischen Lösungsmittels wie Aceton.

In der zweiten Stufe wird die Verbindung V zur Verbindung VI verseift. Die Verseifung kann in bekannter Weise unter Verwendung eines Alkalimetallhydroxids, vorzugsweise von Natriumhydroxid, durchgeführt werden.

Die Säure der Formel VI wird dann zu einer Verbindung der Formel VII decarboxyliert und cyclisiert. Die Decarboxylierung und Cyclisierung kann in bekannter Weise durchgeführt werden, z. B. durch Behandlung mit Essigsäureanhydrid und Natriumacetat bei erhöhter Temperatur.

Schließlich wird die Verbindung der Formel VII durch Chlorierung oder Bromierung in an sich bekannter Weise in das Ausgangsmaterial IIa übergeführt, beispielsweise unter Verwendung von N-Chlorsuccinimid oder, vorzugsweise, N-Bromsuccinimid, in Tetrachlorkohlenstoff bei erhöhter Temperatur unter Bestrahlung.

Die Ausgangsverbindungen der Formel II mit A = Gruppe der Formel (b) können beispielsweise durch Chlorierung von 2-Methyl-5-chlorbenzofuran hergestellt werden. Die Chlorierung bzw. Bromierung des bekannten 2-Methyl-5-chlorbenzofurans kann in Analogie zu der vorstehend bereits beschriebenen Überführung einer Verbindung VII in ein Ausgangsmaterial IIa erfolgen.

Die Alkohole der Formel III sind bekannte Verbindungen.

Die Verbindungen der Formel I enthalten ein asymmetrisches Kohlenstoffatom und können daher in optisch aktiver Form oder als Racemate vorliegen. Die vorliegende Erfindung betrifft daher die Verbindungen der Formel I sowohl in Form optisch aktiver Verbindungen wie in Form von Racematen. Gewünschtenfalls kann ein Racemat in die optischen Antipoden nach an sich bekannten Methoden gespalten werden, beispielsweise durch fraktionierte Kristallisation der mit optisch aktiven Säuren gebildeten Salze.

Die Verbindungen der Formel I können in pharmazeutisch verträgliche Säureadditionssalze durch Behandlung mit einer pharmazeutisch verträglichen anorganischen Säure (z. B. Chlorwasserstoffsäure, Bromwasserstoffsäure, Schwefelsäure, Salpetersäure) oder einer pharmazeutisch verträglichen organischen Säure (z. B. Essigsäure, Zitronensäure, Maleinsäure, Äpfelsäure, Fumarsäure, Bernsteinsäure, Methansulfonsäure, p-Toluolsulfonsäure) übergeführt werden.

Die erfindungsgemäßen Verbindungen besitzen antimykotische Aktivität und können daher als antimykotische Mittel verwendet werden. Sie sind wirksam gegen eine Vielzahl von Pilzen, die topische und systemische Infektionen sowie Infektionen der Schleimhäute hervorrufen. Sie sind beispielsweise wirksam gegen Candida albicans, Tricophyton mentagrophytes, Epidermophyton floccosum, Microsporum canis, Histoplasma capsulatum, Madurella mycetomi und Tricophyton quinckeanum.

Die antimykotische Aktivität der Imidazolderivate kann in vitro durch Behandlung von Standardkulturen verschiedener Mikroorganismen auf Agar-Platten mit den zu testenden Verbindungen bei verschiedenen Konzentrationen gezeigt werden. Die so behandelten Agar-Platten werden 7 Tage bei 37° C inkubiert. Anschließend werden die minimalen Hemmkonzentrationen (MIC) bestimmt. In diesem Test hat beispielsweise 1-[2,4-Dichlor-$\beta$-[(4,6-dichlorbenzofuran-3-yl)methoxy]phenethyl]imidazolnitrat eine MIC (µg/ml) von 10 gegenüber Candida albicans, 3 gegenüber Trichophyton mentagrophytes und weniger als 1 gegen Epidermophyton floccosum und Microsporum canis.

Die antimykotische Aktivität der Imidazolderivate in vivo kann beispielsweise dadurch gezeigt werden, daß man ovarektomierte Ratten, die mit Oestrogenen behandelt werden, vaginal mit Hefezellen von Candida albicans infiziert und dann die Testsubstanzen in verschiedenen Konzentrationen in Form eines Crèmes in Polyethylenglykol intravaginal appliziert. Die zu testenden Verbindungen werden in dieser Weise zweimal täglich an drei aufeinanderfolgenden Tagen, beginnend 24 Stunden nach der Infektion, verabreicht. Von jedem Tier werden am zweiten, vierten und siebten Tag nach der

Infektion Kulturen entnommen, die wachsenden Kolonien werden gezählt, und die Reduktion der Anzahl der Kolonien wird festgestellt im Vergleich mit Kontrolltieren, die ähnlich infiziert aber nur mit Polyethylenglykol behandelt wurden. Die erhaltenen Ergebnisse in diesem Test mit zwei repräsentativen erfindungsgemäßen Verbindungen (Verbindungen A und B) und mit zwei bekannten Imidazolen (Verbindungen C und D) sind in der folgenden Tabelle zusammengefaßt:

Tabelle

| Verbindung | $ED_{50}$ (Konzentration im Crème) |
|---|---|
| A | 0,03% |
| B | 0,07% |
| C | 1,0% |
| D | 0,25% |

Verbindung A = 1-[2,4-Dichlor-$\beta$-[(4,6-dichlorbenzofuran-3-yl)methoxy]phenethyl]imidazolnitrat.
Verbindung B = 1-[2,4-Dichlor-$\beta$-[(5-chlorbenzofuran-2-yl)methoxy]phenethyl]imidazolnitrat.
Verbindung C = 1-[2,4-Dichlor-$\beta$-[(2,4-dichlorbenzyl)-oxy]phenethyl]imidazol (Miconazol).
Verbindung D = 1-[2,4-Dichlor-$\beta$-[(2-chlor-3-thienyl)-methoxy]phenethyl]imidazol (Tioconazol).

Die erfindungsgemäßen Verbindungen können als Medikamente in Form pharmazeutischer Präparate, die sie zusammen mit verträglichen pharmazeutischen Trägermaterialien enthalten, verwendet werden. Beim Trägermaterial kann es sich um ein organisches oder anorganisches inertes Trägermaterial, das für enterale (z. B. orale), topische oder parenterale Administration geeignet ist, handeln, wie Wasser, Gelatine, Talk, Lactose, Stärke, Gummi arabicum, Magnesiumstearat, Polyalkylenglykole oder Vaseline. Die pharmazeutischen Präparate können in fester Form, z. B. als Tabletten, Dragées, Suppositorien, Kapseln; in halbfester Form, z. B. als Salben; oder in flüssiger Form, z. B. als Lösungen, Suspensionen oder Emulsionen, vorliegen. Gegebenenfalls sind sie sterilisiert und bzw. oder enthalten weitere Hilfsstoffe wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Präparate können auch noch andere therapeutisch aktive Substanzen enthalten. Die Herstellung der Präparate kann in der jedem Fachmann geläufigen Weise erfolgen.

Die erfindungsgemäßen Verbindungen können an Erwachsene bei oraler Applikation in Mengen von etwa 2 mg/kg bis etwa 200 mg/kg und bei parenteraler Applikation in Mengen von etwa 0,5 mg/kg bis etwa 50 mg/kg verabreicht werden. Von diesen Mengen kan jedoch auf Verordnung des Arztes sowohl nach oben wie nach unten abgewichen werden, und zwar je nach der zum Einsatz kommenden Verbindung, der Art und Schwere der zu behandelnden Pilzinfektionen sowie den persönlichen Erfordernissen des Patienten.

## Beispiel 1

Eine auf 0° C gekühlte Lösung von 0,51 g (0,002 Mol) 2,4-Dichlor-$\alpha$-[(1-imidazolyl)methyl]benzylalkohol wurde mit 48 mg (0,002 Mol) Natriumhydrid (60 mg einer 80%igen Dispersion in Mineralöl) behandelt. Es wurde weiter gerührt, und nach Beendigung der Gasentwicklung (ca. 30 Minuten) wurde das Gemisch mit einer Lösung von 0,56 g (0,002 Mol) 3-Brommethyl-4,6-dichlorbenzofuran in 2 ml trockenem Dimethylformamid versetzt. Das Gemisch wurde eine halbe Stunde bei 0° C und dann zwei Stunden bei Raumtemperatur gerührt. Nach Zusatz von 50 ml Wasser, das 0,5 ml Eisessig enthielt, und Sättigung mit Natriumchlorid wurde dreimal mit je 30 ml Ethylacetat extrahiert. Die vereinigten Ethylacetatextrakte wurden mit Kochsalzlösung gewaschen, über Natriumsulfat getrocknet und bei 30° C/ 12 mm Hg zu einem braunen viskosen Öl eingeengt. Dieses Öl wurde in 15 ml trockenem Diethylether unter Zusatz einer minimalen Menge Ethylacetat gelöst und mit 0,2 ml konzentrierter Salpetersäure behandelt. Der Niederschlag wurde aus Ethanol umkristallisiert und lieferte 0,57 g 1-[2,4-Dichlor-$\beta$-[(4,6-dichlorbenzofuran-3-yl)methoxy]phenethyl]imidazolnitrat, Smp. 105° C.

Der als Ausgangsmaterial verwendete 2,4-Dichlor-$\alpha$-[(imidazolyl)methyl]benzylalkohol kann nach der Methode von Godefroi et al., J. Med. Chem. 12, 784 (1969), hergestellt werden.

Das als Ausgangsmaterial verwendete 3-Brommethyl-4,6-dichlorbenzofuran kann folgendermaßen hergestellt werden:

(a) Ein Gemisch von 10,25 g (0,05 Mol) 2-Acetyl-3,5-dichlorphenol, 8,05 g (0,05 Mol) Bromessigsäureethylester und 6,91 g (0,05 Mol) Kaliumcarbonat wurden in 150 ml Aceton drei Stunden unter Rückfluß erhitzt. Das Gemisch wurde filtriert und der Rückstand mit 50 ml Aceton gewaschen. Die vereinigten Filtrate wurden zu einem viskosen Öl eingeengt, das in 200 ml Methylenchlorid gelöst und mit Wasser gewaschen wurde. Die organische Phase wurde über das Natriumsulfat getrocknet und zu einem viskosen roten Öl eingeengt, das durch Destillation gereinigt wurde. Man erhielt 13,1 g 2-(2-Acetyl-3,5-dichlorphenoxy)essigsäureethylester, $Kp._{2\,mm\,Hg}$ 180°—182°.

(b) Eine Lösung von 17,4 g (0,06 Mol) 2-(2-Acetyl-3,5-dichlorphenoxy)essigsäureethylester in 300 ml Ethanol wurde mit einer Lösung von 4,8 g (0,12 Mol) Natriumhydroxid in 100 ml Wasser versetzt. Das Gemisch wurde 18 Stunden bei Raumtemperatur stehengelassen, das Ethanol wurde durch Abdampfen entfernt und die verbleibende Lösung mit 6 N HCl angesäuert. Das Gemisch wurde dreimal mit je 75 ml Diethylether extrahiert, die vereinigten Extrakte wurden mit Kochsalzlösung gewaschen, über Natriumsulfat getrocknet und zu 16,4 g eines Rohproduktes eingeengt. Umkristallisation aus Methylenchlorid/Benzol lieferte 14,4 g reine 2-(2-Acetyl-3,5-dichlorphenoxy)essigsäure, Smp. 110° C.

(c) Ein Gemisch aus 5,0 g (0,019 Mol) 2-(2-Acetyl-3,5-dichlorphenoxy)essigsäure, 10,0 g wasserfreiem Natriumacetat und 50 ml Essigsäureanhydrid wurde 30 Minuten auf 155° C erhitzt. Das Gemisch wurde auf 100 g Eis und 100 mg einer gesättigten Natriumbicarbonatlösung gegossen und anschließend dreimal mit je 100 ml Diethylether extrahiert. Die vereinigten Extrakte wurden mit gesättigter Natriumbicarbonatlösung neutral gewaschen, über Natriumsulfat getrocknet und eingeengt. Das Rohprodukt wurde durch Destillation gereinigt und lieferte 2,4 g reines 4,6-Dichlor-3-methylbenzofuran, $Kp._{1\,mm\,Hg}$ 85° C. Das destillierte Produkt wurde aus Petrolether (Kp. 40—60° C) umkristallisiert und lieferte weiße Nadeln mit einem Schmelzpunkt von 45° C.

(d) Eine Lösung von 2,01 g (0,01 Mol) 4,6-Dichlor-3-methylbenzofuran in 50 ml Tetrachlorkohlenstoff wurde mit 1,79 g (0,1 Mol) N-Bromsuccinimid behandelt und unter Bestrahlung mit einer 200-Watt-Lampe unter Rückfluß erhitzt. Nach 1 1/2 Stunden war die Umsetzung beendet (Bestimmung nach Dünnschichtchromatographie). Die Lösung wurde nach Abkühlen filtriert, das Filtrat mit 50 ml Natriumbicarbonatlösung gewaschen, über Natriumsulfat getrocknet und zu einem braunen Öl eingeengt, welches in einem Kugelrohr destilliert wurde. Es wurden 2,2 g eines crèmefarbenen Öls erhalten, das langsam kristallisierte. Umkristallisation aus Petroläther (Kp. 40° —60° C) bei 0° C lieferte 3-Brommethyl-4,6-dichlorbenzofuran in Form crèmefarbiger Nadeln, Smp. 43° C.

## Beispiel 2

In zu Beispiel 1 analoger Weise, aber unter Verwendung von 3-Brommethyl-5,7-dichlorbenzofuran anstelle von 3-Brommethyl-4,6-dichlorbenzofuran, wurde 1-[2,4-Dichlor-β-[(5,7-dichlorbenzofuran-3-yl)methoxy]phenethyl]imidazolnitrat, Smp. 132° C, erhalten.

Das als Ausgangsmaterial verwendete 3-Brommethyl-5,7-dichlorbenzofuran wurde aus 2-Acetyl-4,6-dichlorphenol in Analogie zu der in Beispiel 1(a)—(d) beschriebenen Methode erhalten. Der Schmelzpunkt beträgt 68° C [aus Petrolether (Kp. 40—60° C)].

## Beispiel 3

In zu Beispiel 1 analoger Weise, aber unter Verwendung von 3-Brommethyl-6-chlorbenzofuran anstelle von 3-Brommethyl-4,6-dichlorbenzofuran, wurde 1-[2,4-Dichlor-β-[(6-chlorbenzofuran-3-yl)methoxy]phenethyl]imidazolnitrat, Smp. 154° C, erhalten.

Das als Ausgangsmaterial verwendete 3-Brommethyl-6-chlorbenzofuran wurde aus 2-Acetyl-5-chlorphenol in Analogie zu der in Beispiel 1(a)—(d) beschriebenen Methode erhalten. Der Schmelzpunkt lag bei 25° C [aus Petrolether (Kp. 40—60° C)].

## Beispiel 4

In Analogie zu der in Beispiel 1 beschriebenen Weise, aber unter Verwendung von 2-Brommethyl-5-chlorbenzofuran anstelle von 3-Brommethyl-4,6-dichlorbenzofuran, wurde 1-[2,4-Dichlor-β-[(5-chlorbenzofuran-2-yl)-methoxy]phenethyl]-imidazolnitrat, Smp. 165° C (aus Ethanol), erhalten.

Das als Ausgangsmaterial verwendete 2-Brommethyl-5-chlorbenzofuran kann folgendermaßen hergestellt werden:

2-Methyl-5-chlorbenzofuran (U.S.P. 2 559 532) wird in Analogie zu der in Beispiel 1(d) beschriebenen Weise zu 2-Brommethyl-5-chlorbenzofuran, Smp. 70° C (aus Petrolether Kp. 40—60° C), bromiert.

Das folgende Beispiel illustriert ein typisches pharmazeutisches Präparat auf der Basis eines erfindungsgemäßen Imidazolderivates:

Beispiel A

Eine Salbe für topische Applikation kann folgende Zusammensetzung haben:

| Bestandteil | Gewichtsteile |
|---|---|
| Imidazolderivat | 2 |
| Propylenglykol | 10 |
| Salbengrundlage | 88 |
| | 100 |

**Patentansprüche für die Vertragsstaaten: BE, CH, DE, FR, GB, IT, LI, LU, NL, SE**

1. Imidazolderivate der allgemeinen Formel

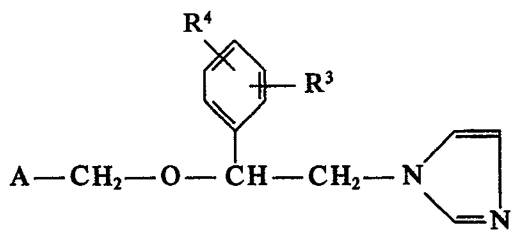

(I)

worin A eine Gruppe der Formel

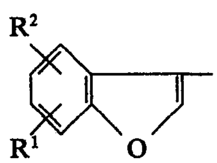

oder

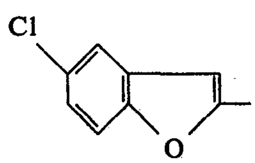

(a) (b)

und $R^1$, $R^2$, $R^3$ und $R^4$ Wasserstoff oder Halogen darstellen,

sowie deren pharmazeutisch verträgliche Säureadditionssalze.

2. Imidazolderivate der Formel I gemäß Anspruch 1, worin A einen Rest der Formel (a) darstellt, und deren pharmazeutisch verträgliche Säureadditionssalze.

3. Imidazolderivate gemäß Anspruch 2, worin $R^1$ Wasserstoff oder Halogen ist und $R^2$, $R^3$ und $R^4$ Halogen darstellen.

4. Imidazolderivate der Formel I gemäß Anspruch 1, worin A einen Rest der Formel (b) und $R^3$ und $R^4$ Halogen darstellen, sowie deren pharmazeutisch verträgliche Säureadditionssalze.

5. 1-[2,4-Dichlor-β-[(4,6-dichlorbenzofuran-3-yl)methoxy]phenethyl]imidazol und dessen pharmazeutisch verträglichen Säureadditionssalze.

6. 1-[2,4-Dichlor-β-[(5,7-dichlorbenzofuran-3-yl)methoxy]phenethyl]imidazol und dessen pharmazeutisch verträgliche Säureadditionssalze.

7. 1-[2,4-Dichlor-β-[(6-chlorbenzofuran-3-yl)methoxy]phenethyl]imidazol und dessen pharmazeutisch verträgliche Säureadditionssalze.

8. 1-[2,4-Dichlor-β-[(5-chlorbenzofuran-2-yl)methoxy]phenethyl]imidazol und dessen pharmazeutisch verträgliche Säureadditionssalze.

9. Eine Verbindung gemäß einem der Ansprüche 1—8 zur Verwendung als antimykotisches Mittel.

10. Verfahren zur Herstellung von Imidazolderivaten gemäß Anspruch 1, dadurch gekennzeichnet, daß man eine Verbindung der Formel

$$A{-}CH_2{-}X \qquad (II)$$

worin A die in Anspruch 1 angegebene Bedeutung hat und X Chlor oder Brom darstellt,

mit einem Alkalimetallsalz eines Alkohols der allgemeinen Formel

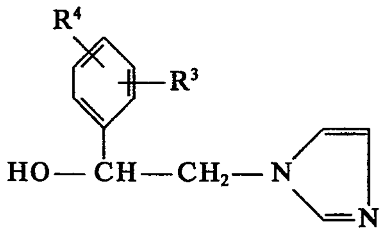

(III)

worin $R^3$ und $R^4$ die in Anspruch 1 angegebenen Bedeutungen haben

umsetzt und, gewünschtenfalls, ein erhaltenes Racemat in die optischen Antipoden auftrennt, und gewünschtenfalls, eine Verbindung der Formel I in ein pharmazeutisch verträgliches Säureadditionssalz überführt.

11. Verfahren gemäß Anspruch 10, dadurch gekennzeichnet, daß als Ausgangsmaterial eine Verbindung der Formel II verwendet wird, in der A eine Gruppe der Formel (a) ist.

12. Pharmazeutische Präparate auf der Basis eines Imidazolderivates gemäß einem der Ansprüche 1—8.

**Patentansprüche für den Vertragsstaat: AT**

1. Verfahren zur Herstellung von Imidazolderivaten der allgemeinen Formel

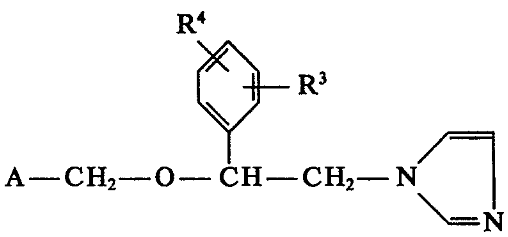

(I)

worin A eine Gruppe der Formel

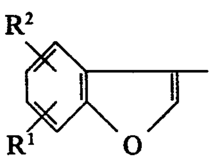

oder

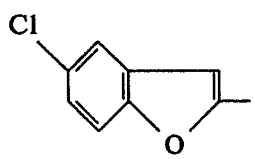

(a) (b)

und $R^1$, $R^2$, $R^3$ und $R^4$ Wasserstoff oder Halogen darstellen,

und von deren pharmazeutisch verträglichen Säureadditionssalzen, dadurch gekennzeichnet, daß man eine Verbindung der allgemeinen Formel

$$A-CH_2-X \qquad (II)$$

worin A die in Anspruch 1 angegebene Bedeutung hat und X Chlor oder Brom darstellt,

mit einem Alkalimetallsalz eines Alkohols der allgemeinen Formel

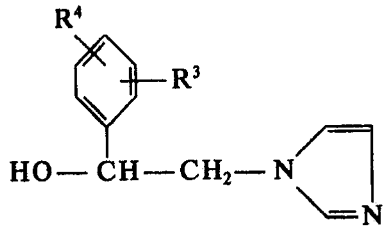

(III)

worin $R^3$ und $R^4$ die oben angegebenen Bedeutungen haben

umsetzt und, gewünschtenfalls, ein erhaltenes Racemat in die optischen Antipoden auftrennt, und gewünschtenfalls, eine Verbindung der Formel I in ein pharmazeutisch verträgliches Säureadditionssalz überführt.

2. Verfahren gemäß Anspruch 1, dadurch gekennzeichnet, daß man als Ausgangsmaterial eine Verbindung der Formel II, in der A eine Gruppe der Formel (a) bedeutet, einsetzt.

3. Verfahren gemäß Anspruch 1, dadurch gekennzeichnet, daß man als Ausgangsmaterial eine Verbindung der Formel II verwendet, in der A eine Gruppe der Formel (b) darstellt.

4. Verfahren gemäß Anspruch 2, dadurch gekennzeichnet, daß man 1-[2,4-Dichlor-$\beta$-[(4,6-dichlorbenzofuran-3-yl)methoxy]phenethyl]imidazol aus 2,4-Dichlor-$\alpha$-[(1-imidazol)methyl]benzylalkohol und 3-Brommethyl-4,6-dichlorbenzofuran herstellt.

**Claims for the Contracting States: BE, CH, DE, FR, GB, IT, LI, LU, NL, SE**

1. Imidazole derivatives of the general formula

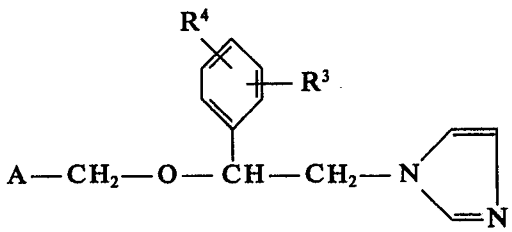

(I)

wherein A represents a group of the formula

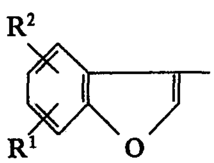

(a)

or

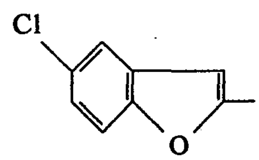

(b)

and $R^1$, $R^2$, $R^3$ and $R^4$ represent hydrogen or halogen,

as well as their pharmaceutically compatible acid addition salts.

2. Imidazole derivatives of formula I in accordance with claim1, wherein A represents a residue of formula (a) and their pharmaceutically compatible acid addition salts.

3. Imidazole derivatives in accordance with claim 2, wherein $R^1$ ist hydrogen or halogen and $R^2$, $R^3$ and $R^4$ represent halogen.

4. Imidazole derivatives of formula I in accordance with claim 1, wherein A represents a residue of formula (b) and $R^3$ and $R^4$ represent halogen as well as their pharmaceutically compatible acid addition salts.

5. 1-[2,4-Dichloro-$\beta$-[(4,6-dichlorobenzofuran-3-yl)methoxy]phenethyl]imidazole an its pharmaceutically compatible acid addition salts.

6. 1-[2,4-Dichloro-$\beta$-[(5,7-dichlorobenzofuran-3-yl)methoxy]phenethyl]imidazole and its pharmaceutically compatible acid addition salts.

7. 1-[2,4-Dichloro-$\beta$-[(6-chlorobenzofuran-3-yl)methoxy]phenethyl]imidazole and its pharmaceutically compatible acid addition salts.

8. 1-[2,4-Dichloro-$\beta$-[(5-chlorobenzofuran-2-yl)methoxy]phenethyl]imidazole and its pharmaceutically compatible acid addition salts.

9. A compound in accordance with any one of claims 1—8 for use as an antimycotic agent.

10. A process for the manufacture of imidazole derivatives in accordance with claim 1, characterized by reacting a compound of the formula

$$A-CH_2-X \qquad (II)$$

wherein A has the significance given in claim 1 and X represents chlorine or bromine,

with an alkali metal salt of an alcohol of the general formula

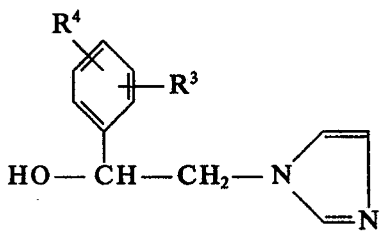

(III)

wherein $R^3$ and $R^4$ have the significances given in claim 1,

and, if desired, resolving a racemate obtained into the optical antipodes and, if desired, converting a compound of formula I into an pharmaceutically compatible acid addition salt.

11. A process in accordance with claim 10, characterized in that a compound of formula II in which A is a group of formula (a) is used as the starting material.

12. Pharmaceutical preparations based on an imidazole derivative in accordance with any one of claims 1—8.

**Claims for the Contracting State: AT**

1. A process for the manufacture of imidazole derivatives of the general formula

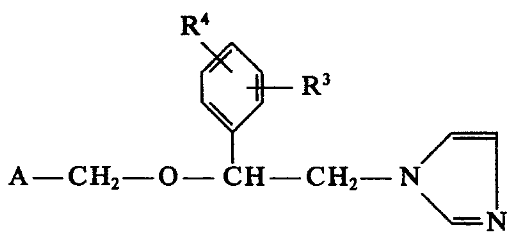

(I)

wherein A represents a group of the formula

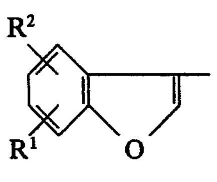

or

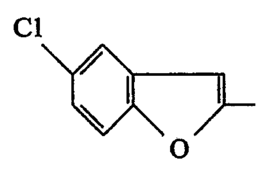

(a) (b)

and $R^1$, $R^2$, $R^3$ and $R^4$ represent hydrogen or halogen,

and of their pharmaceutically compatible acid addition salts, characterized by reacting a compound of the general formula

$$A-CH_2-X \qquad (II)$$

wherein A has the sinificance given above and X represents chlorine or bromine,

with an alkali metal salt of an alcohol of the general formula

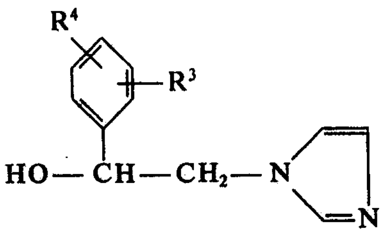

(III)

wherein $R^3$ and $R^4$ have the significances given above,

and, if desired, resolving a racemate obtained into the optical antipodes and, if desired, converting a compound of formula I into a pharmaceutically compatible acid addition salt.

2. A process in accordance with claim 1, characterized in that a compound of formula II in which A signifies a group of formula (a) is used as the starting material.

3. A process in accordance with claim 1, characterized in that a compound of formula II in which A represents a group of formula (b) is used as the starting material.

4. A process in accordance with claim 2, characterized in that 1-[2,4-dichloro-$\beta$-[(4,6-dichlorobenzofuran-3-yl)methoxy]phenethyl]imidazole is manufactured from 2,4-dichloro-$\alpha$-[(imidazolyl)methyl]-benzyl alcohol and 3-bromomethyl-4,6-dichlorobenzofuran.

**Revendications pour les Etats contractants: BE, CH, DE, FR, GB, IT, LI, LU, NL, SE**

1. Dérivés d'imidazole de formula générale

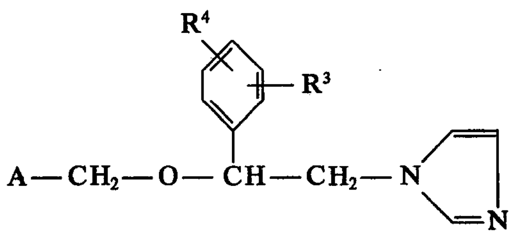

(I)

où A représente un groupe der formule

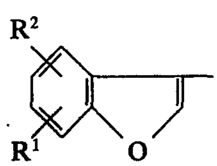

ou

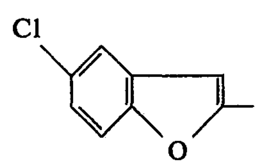

(a) (b)

et $R^1$, $R^2$, $R^3$ et $R^4$ représentent un hydrogène ou un halogène,

ainsi que leurs sels d'addition d'acides pharmaceutiquement acceptables.

2. Dérivés d'imidazole de formule I selon la revendication 1, où A représente un radical de formula (a) et leurs sels d'addition d'acides pharmaceutiquement acceptables.

3. Dérivés d'imidazole selon la revendication 2, où $R^1$ représente un hydrogène ou un halogène et $R^2$, $R^3$ et $R^4$ représentent un halogène.

4. Dérivés d'imidazole de formule I selon la revendication 1, où A représente un radical de formula (b) et $R^3$ et $R^4$ représentent un halogène ainsi que leurs sels d'addition d'acides pharmaceutiquement acceptables.

5. 1-[2,4-dichloro-$\beta$-[(4,6-dichlorobenzofuran-3-yl)méthoxy]phénéthyl]imidazole et ses sels d'addition d'acides pharmaceutiquement acceptables.

6. 1-[2,4-dichloro-$\beta$-[(5,7-dichlorobenzofuran-3-yl)méthoxy]phénéthyl]imidazole et ses sels d'addition d'acides pharmaceutiquement acceptables.

7. 1-[2,4-dichloro-$\beta$-[(6-chlorobenzofuran-3-yl)méthoxy]phénéthyl]imidazole et ses sels d'addition d'acides pharmaceutiquement acceptables.

8. 1-[2,4-dichloro-$\beta$-[(5-chlorobenzofuran-2-yl)méthoxy]phénéthyl]imidazole et ses sels d'addition d'acides pharmaceutiquement acceptables.

9. Composé selon l'une des revendications 1 à 8 aux fins d'application comme agent antimycotique.

10. Procédé de préparation de dérivés d'imidazole selon la revendication 1, caractérisé en ce qu'on fait réagir un composé de formule

$$A-CH_2-X \qquad (II)$$

où A a la signification donnée dans la revendication 1 et X représente un chlore ou un brome,

avec un sel de métal alcalin d'un alcool de formule générale

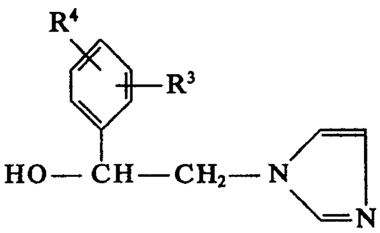

(III)

où $R^3$ et $R^4$ ont les significations données dans la revendication 1,

et, si on le désire, en ce qu'on dédouble un racémate obtenu pour donner les antipodes optiques et, si on le désire, en ce qu'on transforme un composé de formule I en un sel d'addition d'acide pharmaceutiquement acceptable.

11. Procédé selon la revendication 10, caractérisé en ce qu'onutilise comme produit de départ un composé de formule II où A est un groupe de formule (a).

12. Préparations pharmaceutiques à base d'un dérivé d'imidazole selon l'une des revendications 1—8.

**Revendications pour l'Etat contractant: AT**

1. Procédé de préparation de dérivés d'imidazole de formule générale

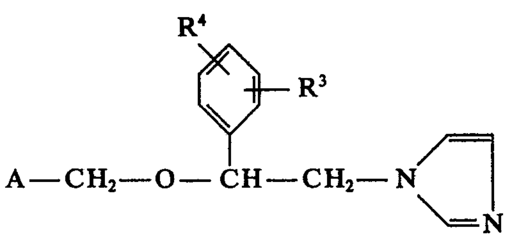

(I)

où A représente un groupe de formule

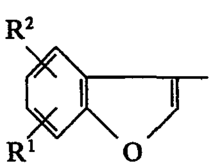

(a)

ou

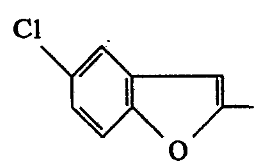

(b)

et $R^1$, $R^2$, $R^3$ et $R^4$ représentent un hydrogène ou un halogène,

et de leurs sels d'addition d'acides pharmaceutiquement acceptables, caractérisé en ce qu'on fait réagir un composé de formule générale

$$A-CH_2-X \qquad (II)$$

où A a la signification donnée ci-dessus et X représente un chlore ou un brome,

avec un sel de métal alcalin d'un alcool de formule générale

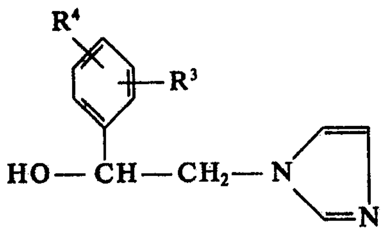

(III)

où $R^3$ et $R^4$ ont les significations données ci-dessus,

et, si on le désire, en ce qu'on dédouble un racémate obtenu pour donner les antipodes optiques et, si on le désire, en ce qu'on transforme un composé de formule I en un sel d'addition d'acide pharmaceutiquement acceptable.

2. Procédé selon la revendication 1, caractérisé en ce qu'on utilise comme produit de départ un composé de formule II où A représente un groupe de formule (a).

3. Procédé selon la revendication 1, caractérisé en ce qu'on utilise comme produit de départ un composé de formule II où A représente un groupe de formule (b).

4. Procédé selon la revendication 2, caractérisé en ce qu'on prépare le 1-[2,4-dichloro-$\beta$-[(4,6-dichlorobenzofuran-3-yl)méthoxy]phénéthyl]imidazole à partir du 2,4-dichloro-$\alpha$-[(1-imidazol)méthyl]benzylalcool et du 3-bromométhyl-4,6-dichlorobenzofurane.